(19) Europäisches Patentamt
European Patent Office
Office européen des brevets

(11) Publication number: 0 001 721 B1

(12) EUROPEAN PATENT SPECIFICATION

(45) Date of publication of patent specification: 16.09.81

(51) Int. Cl.³: C 07 C 121/78, C 07 C 103/365, A 01 N 37/18, A 01 N 37/34

(21) Application number: 78300544.0

(22) Date of filing: 25.10.78

# (54) N-cyano- and N-alkynyl-2-(substituted phenoxy)butyramides and the use thereof as mildewicides.

(30) Priority: 26.10.77 US 845513
26.10.77 US 845514

(43) Date of publication of application:
02.05.79 Bulletin 79/9

(45) Publication of the grant of the European patent:
16.09.81 Bulletin 81/37

(84) Designated Contracting States:
BE CH DE FR GB NL

(56) References cited:
US - A - 3 557 209
US - A - 3 932 168
US - A - 3 971 850
US - A - 4 001 427
US - A - 4 051 184
US - A - 4 052 432

(73) Proprietor: STAUFFER CHEMICAL COMPANY
Westport Connecticut 06880 (US)

(72) Inventor: Walker, Francis Harry
302 Montford Avenue
Mill Valley California 94941 (US)
Inventor: Baker, Don Robert
15 Muth Drive
Orinda California 94563 (US)

(74) Representative: Smith, Sydney et al,
Elkington and Fife High Holborn House 52/54
High Holborn
London WC1V 6SH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

N-cyano- and N-alkynyl-2-(substituted phenoxy) butyramides and the use thereof as mildewicides

This invention relates to N-cyano and N-alkynyl-2-(substituted phenoxy) butyramides which are useful as mildewicides.

In U.S. Patent No. 3,557,209 there are disclosed compounds of the general formula:—

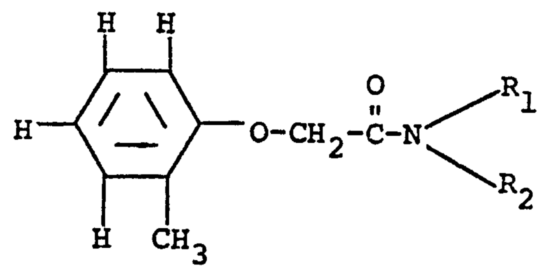

wherein $R_1$ is selected from the group consisting of lower alkyl, lower alkenyl, lower alkynyl, haloalkyl, haloalkenyl, hydroxyalkyl, carboxyalkyl, lower alkoxyalkyl and cyanoalkyl; and $R_2$ is selected from the group consisting of hydrogen, lower alkyl, lower alkenyl, lower alkynyl, haloalkyl, haloalkenyl, hydroxyalkyl, carboxyalkyl, lower alkoxyalkyl and cyanoalkyl.

The use of these compounds as herbicides is disclosed.

In U.S. Patent No. 4,001,427 there are disclosed compounds of the general formula:—

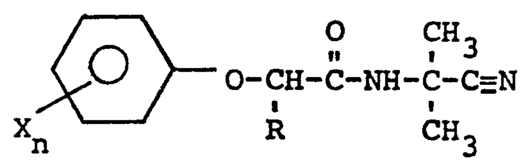

wherein R is either methyl or ethyl, X is either chlorine of trifluoromethyl, and n is either 1 or 3.

The use of these compounds as miticides is disclosed.

Finally, in U.S. Patent No. 4,051,184, there are disclosed compounds of the general formula:—

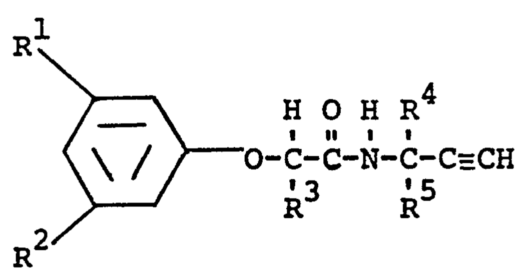

in which $R^1$ and $R^2$ are independently halogen or alkyl having 1 to 4 carbon atoms; $R^3$ is alkyl having 1 to 4 carbon atoms; $R^4$ and $R^5$ are independently hydrogen or alkyl having 1 to 3 carbon atoms.

The use of these compounds as herbicides is disclosed.

The present invention provides compounds of the following general formula:—

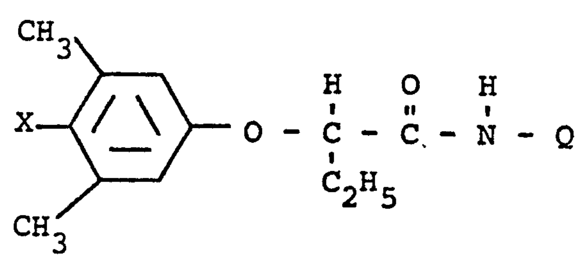

wherein

Q represents 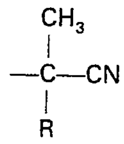 or 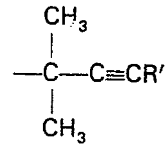 ;

when Q represents 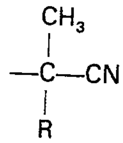

X represents chlorine, bromine, thiocyano or methyl; and R represents methyl, ethyl or methoxymethyl; and when Q represents

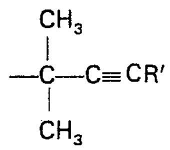

X represents bromine or methyl; and R′ represents hydrogen or methyl. These compounds have activity as mildewicides.

The compounds according to the invention differ from the compounds of the prior art in the substitution in the phenoxy moiety. They also differ in their utility.

In one embodiment of the present invention there are provided compounds corresponding to the following general formula:—

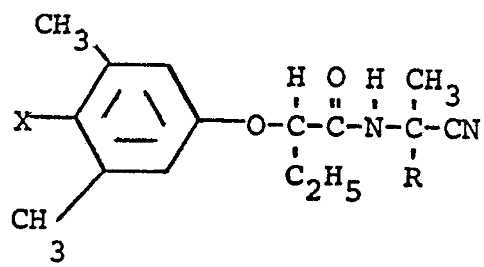

wherein X represents chlorine, bromine, thiocyano or methyl; and R represents methyl, ethyl or methoxymethyl preferably represents chlorine, bromine or methyl, most preferably chlorine or methyl. R preferably represents methyl.

In another embodiment of the present invention there are provided compounds corresponding to the following general formula:

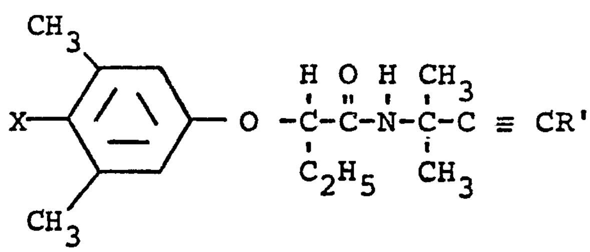

wherein X represents bromine or methyl; and R′ represents hydrogen or methyl.

X preferably represents bromine. R preferably represents methyl.

The present compounds may be prepared by conventional processes.

The present invention further provides a composition which comprises such a compound and an inert carrier or diluent.

The present invention also provides a method for controlling mildew which comprises applying to the locus where the said control is desired such a compound or such a composition.

As indicated above, the present compounds have a utility as mildewicides for controlling the growth of mildew when used in a mildewicidally effective amount.

The term "mildewicide", as used herein, refers to a compound which is useful for controlling the growth of fungi, referred to as mildew. By the term "controlling" is meant the prevention of the growth of the mildew fungi to be controlled.

The present compounds may, for example, be prepared as follows:

(1) *Preparation of a 3,4,5-trisubstituted phenoxy alkanoic acid.*

A 3,4,5-trisubstituted phenol is reacted with a halo-substituted aliphatic acid corresponding to the following general formula:

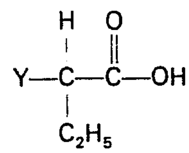

wherein Y represents Cl or Br; in the presence of sodium hydroxide at a temperature of from 40 to 100°C to give the corresponding 2-(3,4,5-trisubstituted) phenoxy alkanoic acid.

(2) *Preparation of a 3,4,5-trisubstituted phenoxy alkanoic acid chloride.*

The acid prepared in step (1) above is reacted with phosgene at a temperature of from 40 to 70°C in the presence of dimethyl formamide as a catalyst to give the corresponding 3,4,5-trisubstituted phenoxy alkanoic acid chloride.

(3) *Preparation of the present 3,4,5-trisubstituted phenoxy alkanoic amides.*

The acid chloride prepared in step (2) above is reacted with an amine corresponding to the following general formula:

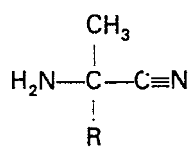

wherein R is as defined above; or an amine corresponding to the following general formula:

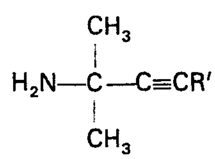

wherein R' is as defined above; in the presence of sodium hydroxide or an organic base, such as triethyl amine, in a solvent, such as methylene chloride, at a temperature of from −15 to +35°C to give the desired amide.

An alternative method of preparation is to react an $\alpha$-halo-substituted aliphatic acid corresponding to the following general formula:

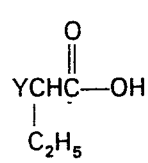

wherein Y represents Cl or Br; with phosgene and dimethyl formamide catalyst to give the corresponding acyl chloride corresponding to the following general formula:

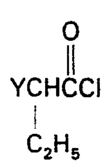

wherein Y is as defined above; which in turn is reacted with an amine corresponding to the following general formula:

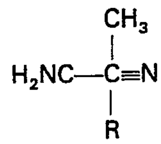

wherein R is as defined above; or an amine corresponding to the following general formula:

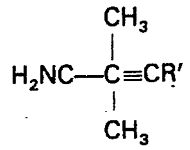

wherein R′ is as defined above; in the presence of an organic base, such as triethylamine, to give the corresponding $\alpha$-haloalkylamide.

This amide is reacted with the sodium salt of a phenol corresponding to the following formula:

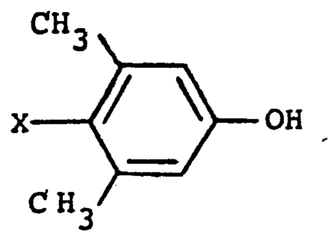

wherein X is as defined above; prepared by the reaction of this phenol and sodium hydride in tetrahydrofuran as solvent to give the present amides.

The following Examples illustrate the present invention.

Example I

*N - dimethylacetonitrilo - 2 - (4 - chloro - 3,5 - dimethylphenoxy) butyramide*

(a) 50 grams (0.32 moles) of 4 - chloro - 3,5 - dimethylphenol were mixed with 58.5 grams (0.35 mol) of 2-bromo-butyric acid in a 500 millilitre flask equipped with stirring equipment maintained at a temperature of 15°C. 60.8 grams (0.76 mole) of 50% aqueous sodium hydroxide were added to the mixture with rapid stirring. The temperature rose to 45°C over the course of the addition and was maintained below 45°C using a cold bath. After all the sodium hydroxide had been added, cooling was terminated and the mixture was heated at 110°C for 15 minutes. Then some water 80 millilitres of perchloroethylene and 65 millilitres of concentrated HCl were added with stirring, the mixture was heated to 85°C, phase-separated and the organic layer was cooled. The product, which was identified by analysis of nuclear magnetic resonance spectra as 2 - (4 - chloro - 3,5 - dimethyl phenoxy) butyric acid, separated as a solid which was removed by filtration and air dried to give 60.7 grams (78% yield) of product having a melting point of 99—102°C.

(b) 57.4 grams (0.24 mole) of the acid produced in step (a) above were slurried in 150 millilitres of toluene in a 500 millilitre flask fitted with a gas-inlet tube, stirrer, thermometer and dry ice/isopropyl alcohol condenser. 0.2 millilitre of dimethyl formamide was added and the mixture was heated to 60°C. Phosgene was passed into the mixture at a moderate rate until 30 grams (0.31 mole) had been added. At the conclusion of the phosgene addition, the dry ice condenser was removed and replaced with a water-cooled condenser. Excess phosgene and hydrogen chloride were removed by purging using argon at 60°C. 60.7 grams (96% yield) of a product, which was identified by analysis of nuclear magnetic resonance spectra as 2 - (4 - chloro - 3,5 - dimethyl phenoxy) butyryl chloride, was recovered as an oil from the solution by cooling the solution and removing the solvent under vacuum.

(c) 8 grams (0.03 mole) of the acyl chloride prepared in step (b) above were added dropwise to a 300 millilitre flask containing a stirred solution of 2.9 grams (0.04 mole $\alpha$-aminoisobytyronitrile and 3.5 grams (0.04 mole) triethylamine in 100 millilitre of methylene chloride at from 10 to 15°C. Some cooling was necessary to maintain the temperature. After all the acyl chloride was added, the mixture was allowed to come to room temperature and the product was isolated by sequentially washing with 100 millilitres each of water, dilute HCl, 5% $Na_2CO_3$ solution and water. The organic phase was dried over magnesium sulphate and the solvent was removed in vacuum to leave 9.0 grams (97% yield) of a solid having a melting point of 108—110°C. The product was identified as the title compound by analysis of nuclear magnetic resonance spectra.

Example II

*N-methyl, methoxymethylacetonitrilo - 2 - (4 - chloro - 3,5 - dimethyl phenoxy) butyramide*

8.0 grams (0.03 mole) of the acyl chloride prepared as in Example I(b) was added dropwise, as in Example I(c), to a stirred mixture of 4.5 grams (0.03 mole) of 1 - cyano - 1 - methyl - 2 - methoxy ethyl amine hydrochloride, 4.8 grams (0.06 mole) 50% aqueous sodium hydroxide, 15 millilitres water and 90 millilitres methylene chloride at from 10 to 15°C. The mixture was allowed to come to room temperature after addition was complete. The material was isolated as in Example I(c) to give 2.0 grams of a solid, which when recrystallized from ethanol, had a melting point of 120—127°C. The product was identified as the title compound by analysis of nuclear magnetic resonance spectra. The yield was 20%.

Example III

*N - dimethylacetonitrilo - 2 - (4 - bromo 3,5 - dimethyl phenoxy) butyramide*

(a) 4 - bromo - 3,5 dimethylphenoxy butyric acid, which was identified by analysis of nuclear magnetic resonance spectra, was prepared as in Example I, but using:

18 grams (0.09 mole) 4-bromo-3,5-dimethylphenol,
18 grams (0.11 mole) 2-bromobutyric acid and
18.4 grams (0.23 mole) 50% NaOH

The only difference in the work-up was that 20 millilitres of water, 50 millilitres of perchloroethylene and 20 millilitres of concentrated HCl were used. 21.3 grams of the acid having a melting point of 78—85°C was produced. Yield was 82% of theoretical value.

(b) 4 - bromo - 3,5 - dimethylphenoxy butyryl chloride was prepared as in Example I(b) but using:

21.3 grams acid (0.07 mole),
10.0 grams phosgene (0.10 mole),
50 millilitres of toluene and
0.2 millilitres of dimethyl formamide

to yield 21.3 grams of the butyryl chloride as an oil.

(c) N - dimethylacetonitrilo - 2 - (4 - bromo - 3,5 - dimethylphenoxy) butyramide, which was identified by analysis of nuclear magnetic resonance spectra, was then prepared as in Example I(c) but using:

80 grams acyl chloride (0.03 mole),
4.0 grams $\alpha$-aminoisobutyronitrile, 85% pure (4.0 mole) and
4.0 grams triethylamine (0.04 mole)

in 100 millilitres of benzene as the reaction solvent to yield 6.5 grams of a solid having a melting point of 83—87°C. The yield was 61% of theoretical value.

Example IV

*N - dimethylacetonitrilo - 2 - (3,4,5 - trimethylphenoxy) butyramide*

(a) 2 - (3,4,5 - trimethylphenoxy) butyric acid, which was identified by analysis of nuclear magnetic resonance spectra, was prepared as in Example I(a) only using:

50 grams (0.37 mole) 3,4,5-trimethylphenol,
74 grams (0.44 mole) 2-bromobutyric acid and
76.1 grams (0.95 mole) 50% aqueous sodium hydroxide.

The product was worked-up in the same manner with:

90 millilitres $H_2O$,
90 millilitres perchloroethylene and
90 millilitres concentrated HCl

51.0 grams of the acid having a melting point of 55—64°C was produced. The yield was 62% of theoretical value.

(b) 2-(3,4,5-trimethylphenoxy) butyryl chloride, which was identified by analysis of nuclear magnetic resonance spectra, was prepared as in Example I(b) only using:

51 grams acid (0.23 mole),
27 grams phosgene (0.28 mole),
150 millilitres toluene and
2 millilitres dry dimethylformamide

to give acyl chloride, 52.0 grams (94% yield) to yield 52.0 grams of the butyryl chloride.

(c) N - dimethylacetonitrilo - 2 - (3,4,5 - trimethylphenoxy) butyramide, which was identified by analysis of nuclear magnetic resonance spectra, was prepared as in Example I(c) but using:

7.2 grams (0.03 mole) acid chloride,
2.5 grams (0.03 mole) $\alpha$-aminoisobutyronitrile,
3.0 grams (0.03 mole) triethylamine and
100 millilitres toluene

as a reaction solvent to yield 5.1 grams of a solid having a melting point of 94—98°C. The yield was 58% of theoretical value.

## Example V

*N - (1,1 - dimethyl - 2' - butyryl) - 2 - (4 - bromo - 3,5 - dimethylphenoxy) butyramide*

(a) 18 grams (0.09 mole) 4 - bromo - 3,5 - dimethylphenol were mixed with 18 grams (0.11 mole) 2-bromobutyric acid to a 500 millilitre flask equipped with stirring equipment maintained at a temperature of 15°C. 18.4 grams (0.23 mole) of 50% aqueous sodium hydroxide were added to the mixture with rapid stirring. The temperature rose to 45°C over the course of the addition and was maintained below 45°C using a cold bath. After all the sodium hydroxide had been added, cooling was terminated and the mixture was heated at 110°C for 15 minutes. Then, 20 millilitres of water, 50 millilitres of perchloroethylene and 20 millilitres of concentrated HCl were added with stirring, the mixture was heated to 85°C, phase separated and the organic layer was cooled. The product, which was identified by analysis of nuclear magnetic resonance spectra as 2 - (4 - bromo - 3,5 - dimethylphenoxy) butyric acid, separated as a solid which was removed by filtration and air dried to give 21.3 grams (82% yield) of product having a melting point of 78—85°C.

(b) 21.3 grams (0.07 mole) of the acid prepared in step (a) above were slurried in 50 millilitres of toluene in a 500 millilitre flask fitted with a gas-inlet tube, stirrer, thermometer and dry ice/isopropyl alcohol condenser. 0.2 millilitre of dimethyl formamide was added and the mixture was heated to 60°C. Phosgene was passed into the mixture at a moderate rate until 10.0 grams (0.10 mole) had been added. At the conclusion of the phosgene addition, the dry ice condenser was removed and replaced with a water-cooled condenser. Excess phosgene and hydrogen chloride were removed by purging using argon at 60°C. 21.3 grams (99.6% yield) of the product identified by analysis of nuclear magnetic resonance spectra as 2 - (4 - bromo - 3,5 - dimethyl phenoxy) butyryl chloride was recovered as an oil from the solution by cooling the solution and removing the solvent under vacuum.

(c) 8 grams (0.03 mole) of the acyl chloride produced in step (b) above were added dropwise to a 300 millilitre flask containing a stirred solution of 3.9 grams (0.04 mole) 4 - amino - 4 - methyl - 2 - butyne and 4.0 grams (0.04 mole) triethylamine in 100 millilitres of methylene chloride at from 10 to 15°C. Some cooling was necessary to maintain the temperature. After all the acyl chloride was added, the mixture was allowed to come to room temperature and the product was isolated by sequentially washing with 100 millilitres each of water, dilute HCl, 5% $Na_2CO_3$ solution and water. The organic phase was dried over magnesium sulphate and the solvent was removed in vacuum to leave 6.8 gram (62% yield) of a solid having a melting point of 81.5—92.5°C. The product was identified as the title compound by analysis of nuclear magnetic resonance spectra.

In the following Tables, the above five examples are listed together with four additional examples which were prepared in a manner analogous to that described above, starting with the appropriate materials. Compound numbers have been assigned and are used through the remainder of the specification.

### *Foliar Fungicide Evaluation Tests*

### *Evaluation for Preventive Action on Bean Powdery Mildew*

A candidate chemical is dissolved in an appropriate solvent and diluted with water containing several drops of Tween 20®, a polyoxyethylene sorbitan monolaurate wetting agent. Test concentrations, ranging from 1000 parts per million downward, are sprayed to runoff on the primary leaves of pinto beans (*Phaseolus vulgaris* L.). After the plants are dry, the leaves are dusted with spores of the powdery mildew fungus (*Erysiphe polygoni* De Candolle) and the plants are retained in the greenhouse until the fungal growth appears on the leaf surface. Effectiveness is recorded as the lowest concentration, in parts per million, which will provide 50% reduction in mycelial formation as compared to untreated, inoculated plants. These values are recorded in Table III.

TABLE III
Preventive Action

| Compound Number | Bean Powdery Mildew |
|---|---|
| 1 | 25 |
| 2 | 5 |
| 3 | 50 |
| 4 | 5 |
| 5 | 500 |
| 6 | 1000 |
| 7 | 100 |
| 8 | 25 |
| 9 | 500 |

The compounds of this invention are generally embodied into a form suitable for convenient application. For example, the compounds can be embodied into pesticidal compositions which are provided in the form of emulsions, suspensions, solution, dusts and aerosol sprays. In general, such compositions will contain, in addition to the active compound, the adjuvants which are found normally in pesticidal preparations. In these compositions, the active compounds of this invention can be employed as the sole pesticide component or they can be used in admixture with other compounds having similar utility. The pesticide compositions of this invention can contain, as adjuvants, organic solvents, such as sesame oil, xylene range solvents, heavy petroleum, etc.; water; emulsifying agents; surface active agents; talc; pyrophyllite; diatomite; gypsum; clays, propellants, such as dichlorodifluoromethane, etc. If desired, however, the active compounds can be applied directly to feed stuffs, seeds, etc., upon which these pests feed. When applied in such a manner, it will be advantageous to use a compound which is not volatile. In connection with the activity of the presently disclosed pesticidal compounds, it should be fully understood that it is not necessary that they be active as such. The purposes of this invention will be fully served if the compound is rendered active by external influences, such as light or by some physiological action which occurs when the compound is ingested into the body of the pest.

TABLE I

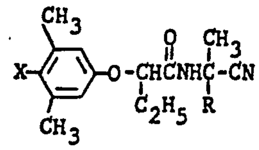

| Compound Number | X | R | Physical Properties |
|---|---|---|---|
| 1. | —Cl | —$CH_3$ | m.p. 108—110°C |
| 2 | —Cl | —$CH_2OCH_3$ | m.p. 120—127°C |
| 3 | —Br | —$CH_3$ | m.p. 83—87°C |
| 4 | —$CH_3$ | —$CH_3$ | m.p. 94—98°C |
| 5 | —Cl | —$CH_2CH_3$ | m.p. 89—93°C |
| 6 | —SCN | —$CH_3$ | m.p. 127—132°C |

TABLE II

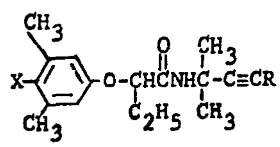

| Compound Number | X | R | Physical Properties |
|---|---|---|---|
| 7 | —Br | —H | m.p. 104—106°C |
| 8 | —Br | —$CH_3$ | m.p. 81.5—82.5°C |
| 9 | —$CH_3$ | —H | m.p. 100—102°C |

The precise manner in which the pesticidal compositions of this invention are used in any particular instance will be readily apparent to a person skilled in the art. Generally, the active pesticide compound will be embodied in the form of a liquid composition; for example, an emulsion, suspension, or aerosol spray. While the concentration of the active pesticide in the present compositions can vary within rather wide limits, ordinarily the pesticide compound will comprise not more than about 15.0% by weight of the composition. Preferably, however, the pesticide compositions of this invention will be in the form of solutions or suspensions containing about 0.1 to about 1.0% by weight of the active pesticide compound.

**Claims**

1. Compounds of the following general formula:—

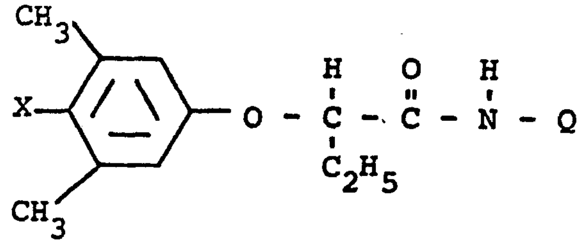

wherein
Q represents 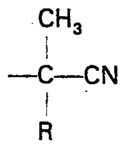 or 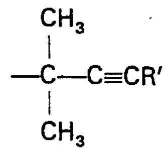

when Q represents 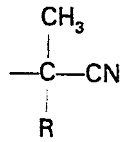

X represents chlorine, bromine, thiocyano or methyl; and R represents methyl, ethyl or methoxymethyl; and when Q represents

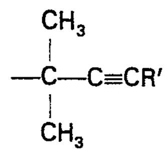

X represents bromine or methyl; and R′ represents hydrogen or methyl.

2. A compound corresponding to the general formula defined in claim 1 wherein X represents chlorine; and R represents methyl.

3. A compound corresponding to the general formula defined in claim 1 wherein X represents chlorine; and R represents methoxymethyl.

4. A compound corresponding to the general formula defined in claim 1 wherein X represents bromine; and R represents methyl.

5. A compound corresponding to the general formula defined in claim 1 wherein X represents methyl; and R represents methyl.

6. A compound corresponding to the general formula defined in claim 1 wherein X represents chlorine; and R represents ethyl.

7. A compound corresponding to the general formula defined in claim 1 wherein X represents thiocyano; and R represents methyl.

8. A compound corresponding to the general formula defined in claim 1 wherein X represents bromine; and R′ represents hydrogen.

9. A compound corresponding to the general formula defined in claim 1 wherein X represents bromine; and R′ represents methyl.

10. A compound corresponding to the general formula defined in claim 1 wherein X represents methyl; and R′ represents hydrogen.

11. A composition which comprises a compound as claimed in any of claims 1 to 10 and an inert carrier or diluent.

12. The use for controlling mildew of a compound as claimed in any of claims 1 to 10 or a composition as claimed in claim 11.

**Revendications**

1. Composés de la formule générale

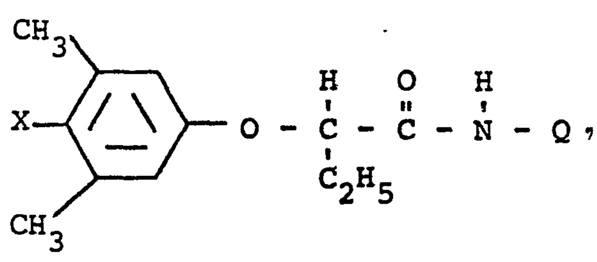

dans laquelle
Q représente un groupe 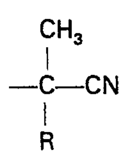 ou 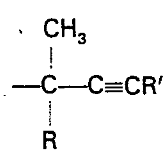

et lorsque Q est un groupe

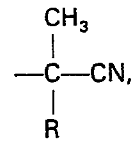

X représente un atome de chlore ou de brome ou un groupe thiocyano ou méthyle et R représente un groupe méthyle, éthyle ou méthoxyméthyle et lorsque Q représente un groupe

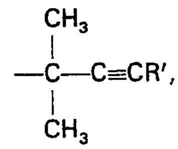

X représente un atome de brome ou un groupe méthoxyméthyle et R' représente un atome d'hydrogène ou un groupe méthyle.

2. Composé de la formule générale définie dans la revendication 1, pour lequel X = —Cl et R = —$CH_3$.

3. Composé de la formule générale définie dans la revendication 1, pour lequel X = —Cl et R = —$CH_2OCH_3$.

4. Composé de la formule générale définie dans la revendication 1, pour lequel X = —Br et R = —$CH_3$.

5. Composé de la formule générale définie dans la revendication 1, pour lequel X = —$CH_3$ et R = —$CH_3$.

6. Composé de la formule générale définie dans la revendication 1, pour lequel X = —Cl et R = —$C_2H_5$.

7. Composé de la formule générale définie dans la revendication 1, pour lequel X = —SCN et R = —$CH_3$.

8. Composé de la formule générale définie dans la revendication 1, pour lequel X = —Br et R' = —H.

9. Composé de la formule générale définie dans la revendication 1, pour lequel X = —Br et R' = —$CH_3$.

10. Composé de la formule générale définie dans la revendication 1, pour lequel X = —$CH_3$ et R' = —H.

11. Composition comprenant un composé suivant l'une quelconque des revendications 1 à 10 et un véhicule ou diluant inerte.

12. Utilisation pour combattre le mildiou d'un composé suivant l'une quelconque des revendications 1 à 10 ou d'une composition suivant la revendication 11.

**Patentansprüche**

1. Verbindungen der folgenden allgemeinen Formel:

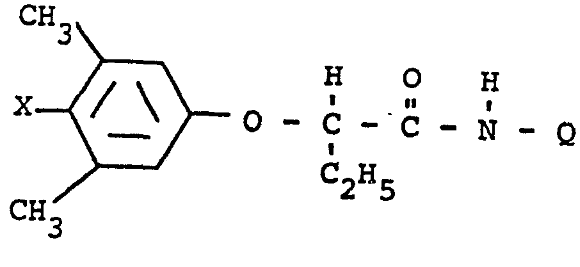

worin Q

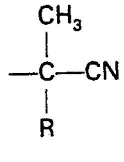

oder

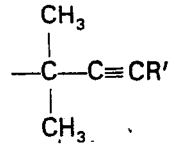

bedeutet, mit der Maßgabe, daß, wenn Q 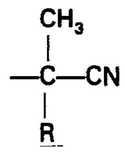

bedeutet, X Chlor, Brom, Thiocyano oder Methyl bedeutet, und R Methyl, Äthyl oder Methoxymethyl bedeutet, und, wenn Q

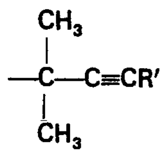

bedeutet, X Brom oder Methyl bedeutet, und R' Wasserstoff oder Methyl bedeutet.

2. Verbindung der allgemeinen Formel, wie sie in Anspruch 1 definiert wurde, dadurch gekennzeichnet, daß X Chlor und R Methyl bedeuten.

3. Verbindung der allgemeinen Formel, wie sie in Anspruch 1 definiert ist, dadurch gekennzeichnet, daß X Chlor und R Methoxymethyl bedeuten.

4. Verbindung der allgemeinen Formel, wie sie in Anspruch 1 definiert ist, dadurch gekennzeichnet, daß X Brom und R Methyl bedeuten.

5. Verbindung der allgemeinen Formel, wie sie in Anspruch 1 definiert ist, dadurch gekennzeichnet, daß X Methyl und R Methyl bedeuten.

6. Verbindung der allgemeinen Formel, wie sie in Anspruch 1 definiert ist, dadurch gekennzeichnet, daß X Chlor und R Äthyl bedeuten.

7. Verbindung der allgemeinen Formel, wie sie in Anspruch 1 definiert ist, dadurch gekennzeichnet, daß X Thiocyano und R Methyl bedeuten.

8. Verbindung der allgemeinen Formel, wie sie in Anspruch 1 definiert ist, dadurch gekennzeichnet, daß X Brom und R' Wasserstoff bedeuten.

9. Verbindung der allgemeinen Formel, wie sie in Anspruch 1 definiert ist, dadurch gekennzeichnet, daß X Brom und R' Methyl bedeuten.

10. Verbindung der allgemeinen Formel, wie sie in Anspruch 1 definiert ist, dadurch gekennzeichnet, daß X Methyl und R' Wasserstoff bedeuten.

11. Mittel, dadurch gekennzeichnet, daß es eine Verbindung nach einem der Ansprüche 1 bis 10 und einen inerten Träger oder ein Verdünnungsmittel enthält.

12. Verwendung einer Verbindung nach einem der Ansprüche 1 bis 10 oder des Mittels nach Anspruch 11 zur Kontrolle von Meltau.